# EUROPEAN PATENT APPLICATION

(11) **EP 3 255 048 A1**
(43) Date of publication of application: **13.12.2017**
(21) Application number: 16173292.0
(22) Date of filing: 07.06.2016
(51) Int. Cl.: C07D 487/04, A61K 31/519, A61P 35/00

(54) **NON-CRYSTALLINE FORM OF PALBOCICLIB**

(71) Applicant: K.H.S. Pharma Holding GmbH, 55218 Ingelheim am Rhein (DE)
(72) Inventor: Haferkamp, Sven, 41063 Mönchengladbach (DE); Kupka, Anna, 44803 Bochum (DE); Bock, Dominique Anna, 45475 Mülheim an der Ruhr (DE); Dworak, Jürgen, 44795 Bochum (DE)
(74) Representative: Drescher, Christian

(57) **Abstract**

The present invention provides a solid non-crystalline form of Palbociclib base, methods of its preparation, and pharmaceutical compositions containing it.

## Description

### Field of the Invention

The present invention relates to a new form of 6-acetyl-8-cyclopentyl-5-methyl-2-[5-(piperazin-1-yl)pyridin-2-ylamino]-8H-pyrido[2,3-d]pyrimidin-7-one of formula (I), 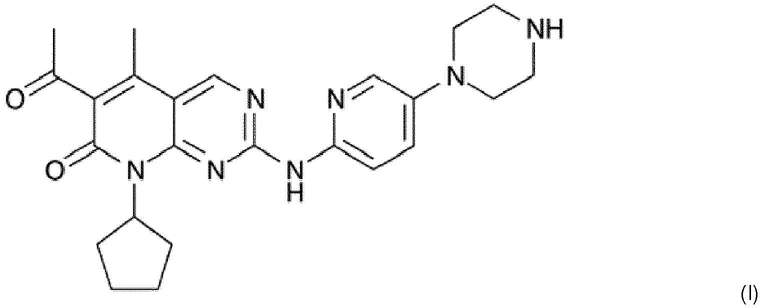 processes for its preparation and pharmaceutical compositions containing the same.

### Background of the Invention

The drug compound 6-acetyl-8-cyclopentyl-5-methyl-2-[5-(piperazin-1-yl)pyridin-2-ylamino]-8H-pyrido[2,3-d]pyrimidin-7-one, also known by its International Nonproprietary Name (INN) Palbociclib, is a cyclin-dependent kinase inhibitor. It is currently registered and marketed under the trade name Ibrance™ for the treatment, in combination with Letrozole, of postmenopausal women with estrogen receptor (ER)-positive, human epidermal growth factor receptor 2 (HER2)-negative advanced breast cancer.

A preparation of Palbociclib is disclosed in WO 2003/062236. Solid crystalline forms of Palbociclib base are first disclosed in international patent application WO 2014/128588. Further crystalline forms of Palbociclib base are disclosed in international patent application WO 2016/024249.

According to US 7,345,171 Palbociclib base has a poor water solubility of only 9µg/mL. According to WO 2014/128588, Palbociclib base prepared by processes already known in the art exhibits poor physicochemical properties.

Therefore, there still remains a demand for additional solid modifications of Palbociclib base with improved properties in a physiological (i.e. aqueous) environment for use in a pharmaceutical formulation. Characteristics like hygroscopicity, solubility, purity and stability can affect the bioavailability of the active pharmaceutical compound. For the preparation of suitable pharmaceutical compositions, mechanical properties such as flowability and processability have to be considered.

### Summary of the Invention

In a first aspect, the present invention provides a new solid form of Palbociclib base. In particular, the present invention relates to a new solid non-crystalline form of Palbociclib base.

In another aspect, the present application discloses methods for the preparation of the new solid, non-crystalline Palbociclib base by means of either precipitation from ionic liquids, cryo grinding or solvent evaporation.

In a further aspect, this invention provides pharmaceutical compositions comprising the newly discovered solid, non-crystalline Palbociclib base, and a process for the preparation of the pharmaceutical composition.

### Brief Description of the Drawings

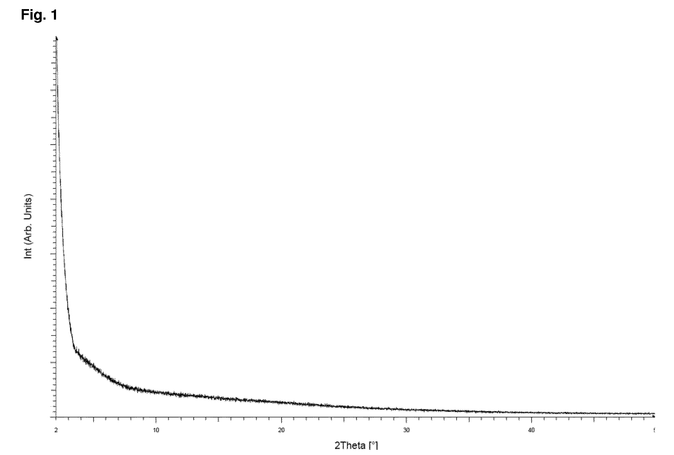

**Figure 1** shows a **PXRD** pattern of non-crystalline Palbociclib base obtained according to the procedure of Example 1 - i.

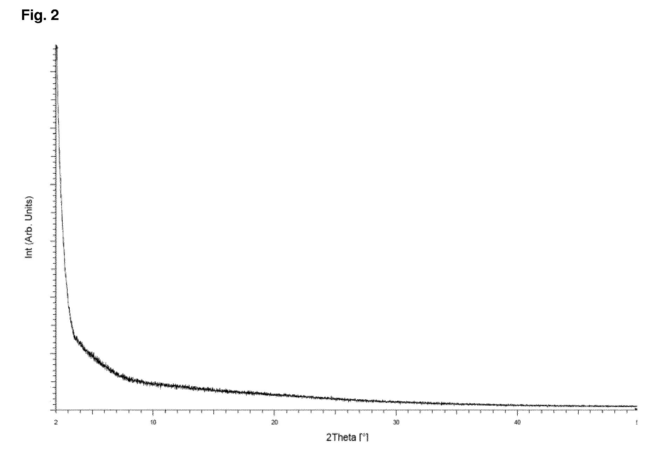

**Figure 2** shows a **PXRD** pattern of non-crystalline Palbociclib base obtained according to the procedure of Example 1 - ii.

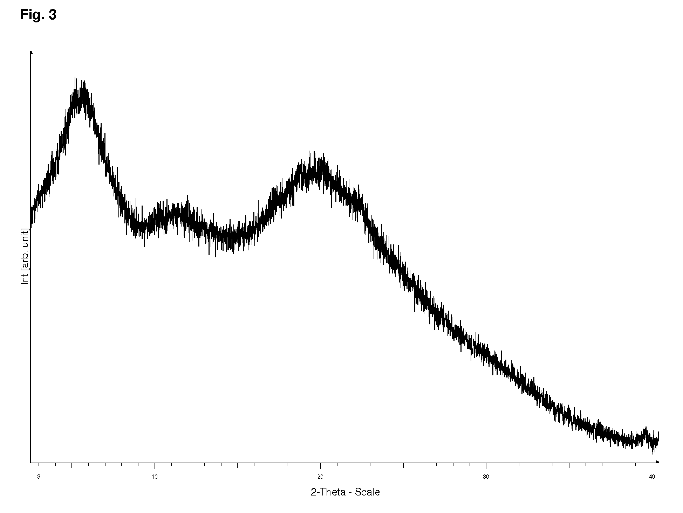

**Figure 3** shows a **PXRD** pattern of non-crystalline Palbociclib base obtained according to the procedure of Example 2.

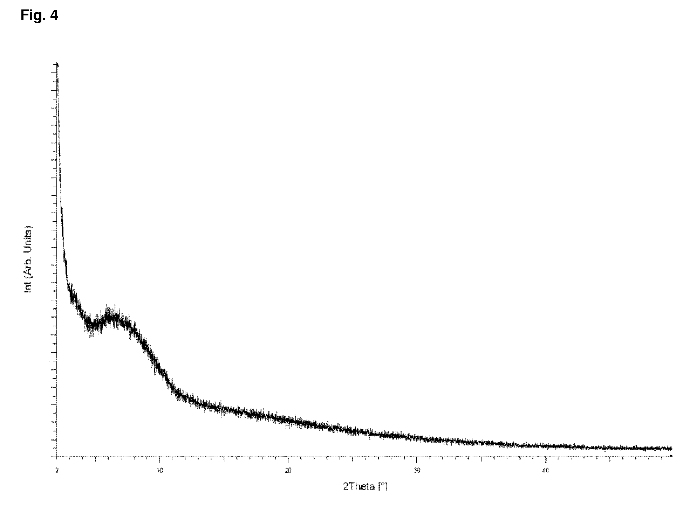

**Figure 4** shows a **PXRD** pattern of non-crystalline Palbociclib base obtained according to the procedure of Example 3.
**Figure 5** shows a comparison of the **PXRD** patterns of Palbociclib base crystalline form A with those obtained according to Examples 1 to 3.

### Detailed Description of the Invention

A first aspect of the invention relates to a new solid form of Palbociclib of formula (I)

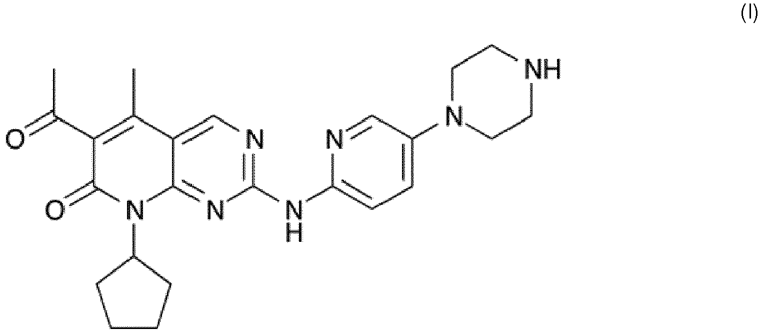

In particular, the present invention relates to a new solid non-crystalline form of Palbociclib base.

Non-crystalline (also called "amorphous") solids offer opportunities for solubility and bioavailability enhancement since these materials are often more soluble than crystalline forms of the same compound.

The components in a non-crystalline substance may not necessarily be arranged completely randomly and purely statistically, but may exhibit a certain regularity and similarity with respect to the orientation of the nearest neighbors, i.e. a somewhat short-range order. Accordingly, non-crystalline substances within the scope of the present invention preferably have a short-range order do but not exhibit any long-range order.

Experimentally, solid non-crystalline forms can be distinguished from crystalline forms by means of X-ray diffraction spectrometry because their diffractograms do not show sharp peaks but only very few diffuse interferences.

Within the scope of the present invention, a "sharp peak" is defined as a discrete signal that does not exhibit a width of more than 1° 2θ.

Accordingly, the non-crystalline Palbociclib base of the present invention may be characterized by a powder X-ray diffraction (PXRD) pattern that does not show any sharp peaks.

The non-crystalline form of Palbociclib base according to the present invention is further characterized by a PXRD pattern which is essentially free from any signals being typical for crystalline forms.

In the context of the present invention, "essentially free" means that the maximum content of any crystalline forms of Palbociclib base is 5.0% by weight. Preferably, the solid non-crystalline Palbociclib base contains less than 3.0% by weight of crystalline Palbociclib base, more preferably less than 1.0% by weight of crystalline Palbociclib base.

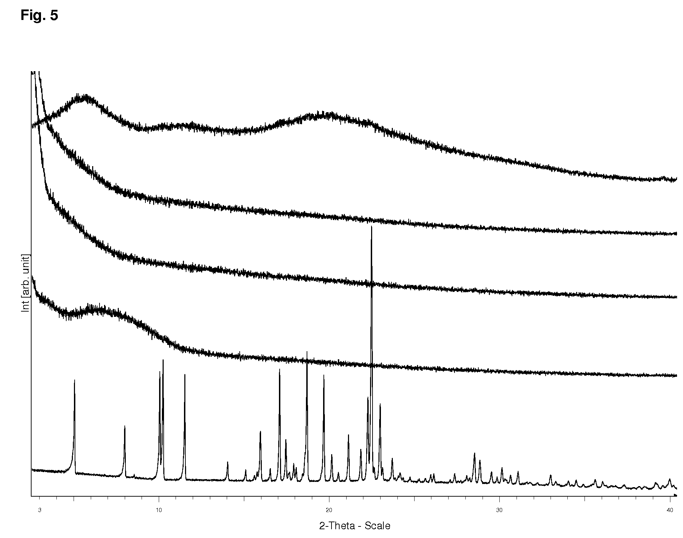

As can be depicted from Fig. 5, the solid non-crystalline Palbociclib base according to the present invention is essentially free from any signals being typical for "Form A" according to international patent application WO 2014/128588, i.e. signals at diffraction angles of 8.0°± 0.2°2θ, 10.10±0.20°2θ, and 11.5°±0.2°2θ.

Accordingly, the solid non-crystalline Palbociclib base according to the present invention contains less than 5.0% by weight, preferably less than 3.0% by weight and more preferably less than 1.0% by weight of Palbociclib base crystalline Form A.

The solid, non-crystalline Palbociclib base according to the present invention can be prepared by several techniques, e.g. precipitation from ionic liquids, cryo grinding and precipitation by solvent evaporation.

The preparation of solid, non-crystalline Palbociclib base by precipitation from ionic liquids comprises the following steps:
- dissolving Palbociclib in an ionic liquid;
- optionally filtering off remaining solids;
- adding an anti-solvent to the filtrate.

The preferred ionic liquids for Palbociclib according to the present invention are 1-hexyl-3-methylimidazolium hexafluorophosphate (PMIM [PF6]) and 1-pentyl-3-methylimidazolium tetrafluoroborate (PMIM [BF4]).
As used herein, the term "anti-solvent" means a liquid which reduces the solubility of Palbociclib upon addition. According to the present invention, the preferred anti-solvent for Palbociclib is water.

Another suitable technique for the preparation of solid, non-crystalline Palbociclib base by cryo grinding comprises the following steps:
- immersing a ball-mill in liquid nitrogen for 5min;
- grinding Palbociclib for up to 120min.

The preparation of solid, non-crystalline Palbociclib base by solvent evaporation comprises the following steps:
- stirring Palbociclib in a poor solvent for 7 days;
- filtering off the solid;
- evaporating the filtrate until dryness
The preferred poor solvent for Palbociclib according to the present invention is water.

The solid, non-crystalline Palbociclib base evaporation according to the present invention may alternatively be obtained by freeze-drying (lyophilisation) or spray drying techniques.

The solid, non-crystalline Palbociclib base according to the present invention may be stabilized via formation of a solid dispersion. Solid dispersions of non-crystalline Palbociclib may be obtained by coprecipitation or extrusion together with pharmaceutically acceptable excipients. Suitable pharmaceutically acceptable excipients include, but are not limited to, polyvinylpyrrolidone, copolymers of N-vinylpyrrolidone, gums, cellulose derivatives including hydroxypropyl methylcellulose (HPMC) and hydroxypropyl cellulose (HPC), cyclodextrins, gelatins, hypromellose phthalate, sugars, polyhydric alcohols and mixtures thereof. Accordingly, this invention further encompasses a pharmaceutical composition comprising solid, non-crystalline Palbociclib base, and at least one pharmaceutically acceptable excipient.

The solid, non-crystalline Palbociclib base according to the present invention shows superior characteristics, thus enlarging the repertoire of materials of the formulation scientist when designing a pharmaceutical dosage form of Palbociclib with a targeted dissolution profile and/or other desired characteristics.

Accordingly, the pharmaceutical compositions containing solid, non-crystalline Palbociclib base of the present invention can be used as a medicament.

### Examples

### Powder X-ray diffractometry

For the powder X-ray diffraction, the samples were filled in standard glass capillaries (Ø = 0.7 mm). Measurements were made at room temperature using a Bruker D8 Advance diffractometer (Johansson primary beam monochromator, position sensitive detector) is carried out in transmission with an axial rotation of the sample.

**Measurement conditions:**

| | |
|---|---|
| 2Theta range | 1.5 - 49.998 2θ |
| Step size | 0.009 2θ |
| Generator kV: | 40 |
| Generator mA | 40 |
| Wavelength type | Cu-Kα₁ (1.54059 Å) |
| Divergence slit | 0.6 mm |
| Antiscatter slit | 2 mm |
| Counts max. | 4000-8000 |
| Measurement time: | 2 hours |

### Example 1: Preparation of non-crystalline Palbociclib base by means of precipitation from ionic liquids

### i. HMIM [PF6]

7.7mg Palbociclib base were dissolved at 60°C in 4.0mL of 1-Hexyl-3-methylimidazolium hexafluorophosphate (PMIM [PF6]) and stirred for 48h. Traces of remaining solid were removed from the obtained saturated solution by filtration. A clear yellowish solution was obtained. After subsequent addition of 3mL of water as antisolvent a phase separation was observed. The (still clear) mixture was stored at T = 5 °C. Within three days, a yellow solid precipitated at the phase boundary.

### ii. PMIM [BF4]

5.9mg Palbociclib base were dissolved at 60°C in 2.3mL of 1-Pentyl-3-methylimidazolium tetrafluoroborate (PMIM [BF4]) and stirred for 48h. Traces of remaining solid were filtered off from the obtained saturated solution. 1mL of water was added as antisolvent to the clear yellowish solution. After phase separation was observed, a yellow solid precipitated at the bottom of the vessel.

### Example 2: Preparation of non-crystalline Palbociclib base by means of cryo grinding

200mg Palbociclib base were ground in a ball mill (Fritsch PULVERISETTE 23) equipped with a steel ball for a total of 120 minutes. Before grinding, the charged mill bowl was immersed in liquid nitrogen for 5min.

### Example 3: Preparation of non-crystalline Palbociclib base by means of solvent evaporation

39.6mg Palbociclib base were stirred with 3.0ml of water for 7 days at 25°C. The solid was filtered off and the filtrate evaporated at 50°C until dryness. A yellowish solid was obtained.

## Claims

1. Solid, non-crystalline form of Palbociclib base.

2. The solid, non-crystalline Palbociclib base according to claim 1, **characterized by** a powder X-ray diffraction (PXRD) pattern that does not show any sharp peaks, in particular no peaks at diffraction angles of 8.0°± 0.2°2θ,10.1°±0.2°2θ, and 11.5°±0.2°2θ.

3. Solid, non-crystalline Palbociclib base comprising less than 5.0%, preferably less than 3.0%, more preferably less than 1.0% by weight of solid crystalline Palbociclib base.

4. The solid, non-crystalline Palbociclib base according to claim 3 wherein the solid crystalline Palbociclib base is Form A with characteristic diffraction angles at 8.0°± 0.2°2θ, 10.1 °±0.2°2θ, and 11.5°±0.2°2θ.

5. A process for the preparation of the non-crystalline Palbociclib base according to any of the preceding claims by cryo-grinding, comprising the steps of
a) Immersing a ball-mill in liquid nitrogen for 5min;
b) grinding Palbociclib for up to 120min.

6. A process for the preparation of the non-crystalline Palbociclib base according to any of the preceding claims 1 to 4 by solvent evaporation, comprising the steps of
a) stirring Palbociclib in a solvent for 7 days;
b) filtering off the solid;
c) evaporating the filtrate until dryness.

7. The process according to claim 6 **characterized in that** the solvent is water.

8. A process for the preparation of the non-crystalline Palbociclib base according to any of the preceding claims 1 to 4 by precipitation from ionic liquids, comprising the steps of
a) dissolving Palbociclib in an ionic liquid;
b) optionally filtering off remaining solids;
c) adding an anti-solvent to the filtrate.

9. The process according to claim 8, **characterized in that** the ionic liquid is selected from organic salts, wherein the cation is selected from the group of, preferably alkylated, imidazolium, pyridinium, pyrrolidinium, guanidinium, uronium, thiouronium, piperidinium, morpholinium, ammonium and phosphonium;
and the anion is selected from the group of hexafluorophosphate [PF6], tetrafluoroborate [BF4], trifluoracetate, triflate, phosphinate and tosylate.

10. The process according to claim 9, **characterized in that** the ionic liquid is one of 1-hexyl-3-methylimidazolium hexafluorophosphate (HMIM [PF6]) or 1-pentyl-3-methylimidazolium tetrafluoroborate (PMIM [BF4]), or mixtures thereof.

11. The process according to any of the preceding claims 8 to 10, **characterized in that** the anti-solvent is water.

12. A pharmaceutical composition comprising non-crystalline Palbociclib base according to any one of the preceding claims, and at least one pharmaceutically acceptable excipient.

13. The pharmaceutical composition according to claim 12 wherein the pharmaceutically acceptable excipient(s) are selected from polyvinylpyrrolidone, copolymers of N-vinylpyrrolidone, gums, cellulose derivatives including hydroxypropyl methylcellulose (HPMC) and hydroxypropyl cellulose (HPC), cyclodextrins, gelatins, hypromellose phthalate, sugars, polyhydric alcohols and mixtures thereof.

14. The pharmaceutical composition according to any of the preceding claims 12 to 13 for use as a medicament.
